(19) Europäisches Patentamt
European Patent Office
Office européen des brevets

(11) Numéro de publication: **0 201 366**
**A1**

# (12) DEMANDE DE BREVET EUROPEEN

(21) Numéro de dépôt: **86400602.8**

(22) Date de dépôt: **21.03.86**

(51) Int. Cl.⁴: **C07D 471/14** , //(C07D471/14,221:00,221:00,209:00)

(30) Priorité: **22.03.85 FR 8504871**

(43) Date de publication de la demande: **17.12.86 Bulletin 86/46**

(84) Etats contractants désignés: **AT BE CH DE FR GB IT LI LU NL SE**

(71) Demandeur: **SANOFI**
**40, Avenue George V**
**F-75008 Paris(FR)**
Demandeur: **CENTRE NATIONAL DE LA RECHERCHE SCIENTIFIQUE (CNRS)**
**13 Quai Anatole France**
**F-75700 Paris(FR)**

(72) Inventeur: **Bisagni, Emile**
**16 rue Bossuet**
**F-91400 Orsay(FR)**
Inventeur: **Chi Hung, Nguyen**
**7 allée des Amonts**
**F-91940 Les Ulis(FR)**
Inventeur: **de Cointet, Paul**
**224 avenue Jean Rieux**
**F-31500 Toulouse(FR)**

(74) Mandataire: **Polus, Camille et al**
**c/o Cabinet Lavoix 2, Place d'Estienne d'Orves**
**F-75441 Paris Cedex 09(FR)**

(54) **Nouvelles 5-H pyrido (3',4':4,5) pyrrolo - (3,2-c) pyridines, leur procédé de préparation et leur application en tant qu'intermédiaires de synthèse.**

(57) La présente invention est relative à de nouveaux dérivés de la 5-H pyrido [3', 4':4,5] pyrrolo [3,2-c] pyridine, de formule

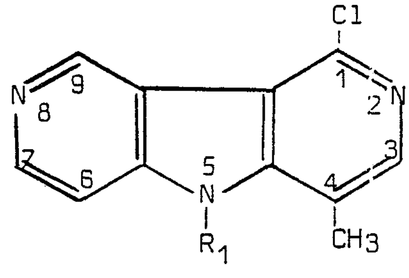

(I)

EP 0 201 366 A1

dans laquelle $R_1$ représente l'hydrogène ou un radical alcoyle inférieur, leur procédé de préparation et leur application en tant qu'intermédiaires de synthèse dans la préparation de nouveaux composés utilisables dans l'industrie pharmaceutique.

"Nouvelles 5-H pyrido [3', 4':4,5] pyrrolo -[3,2-c] pyridines, leur procédé de préparation et leur application en tant qu'intermédiaires de synthèse"

L'invention est relative à de nouveux dérivés de la 5-H pyrido [3', 4' : 4,5] pyrrolo [3,2-c] pyridine, à un procédé pour les préparer et à leur utilisation en tant qu'intermédiaires de synthèse dans la préparation de composés utiles dans l'industrie pharmaceutique.

Les nouveaux dérivés de l'invention répondent à la formule suivante :

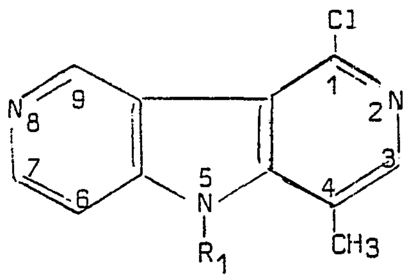

(I)

dans laquelle $R_1$ représente l'hydrogène ou un radical alcoyle inférieur. L'invention concerne aussi les formes tautomères lorsqu'elles existent.

Par radical alcoyle inférieur, on entend une chaîne hydrocarbonée saturée en $C_1$ -$C_4$ linéaire ou ramifiée.

Ces dérivés sont des intermédiaires dans la préparation de nouveaux composés de formule

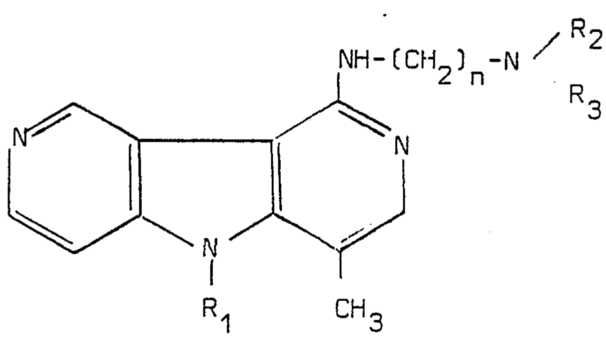

(A)

dans laquelle n représente un nombre entier de 2 à 4, $R_1$ représente l'hydrogène ou un groupe alcoyle inférieur, $R_2$ et $R_3$ sont chacun indépendamment l'un de l'autre l'hydrogène, un groupe alcoyle ou hydroxyalcoyle.

Ces nouveaux composés qui sont doués de propriétés antitumorales intéressantes ont été revendiqués par les demandeurs ainsi que leur préparation et leur application en thérapeutique, dans une demande déposée conjointement sous le N° 85.04872.

L'invention a également pour objet un procédé de préparation des composés de formule (I) ci-dessus caractérisé en ce que

1°) par réaction de l'hydrazine hydratée sur l'hydroxy-4 méthyl-5 1-H pyridone-2, on prépare l'hydrazino-4 pyridone-2 de formule (II)

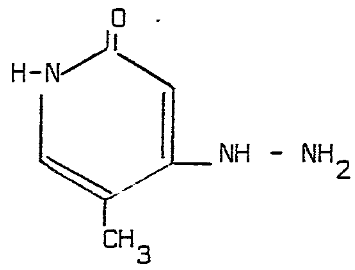

(II)

2°) on fait réagir l'hydrazine de formule (II) sur la N-acétyl pipéridone-4 pour obtenir l'hydrazone correspondante de formule (III)

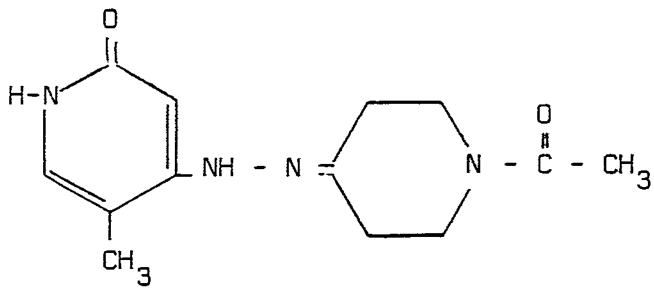

(III)

3°) par réaction de Fisher, on cyclise l'hydrazone de formule (III) pour former la tétrahydro-6,7,8,9 acétyl-8 méthyl-4 2-H,5-H pyrido [3', 4' : 4,5] pyrrolo [3,2-c] pyridone-1 de formule (IV)

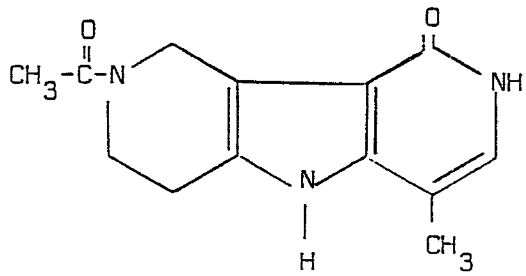

(IV)

4°) on aromatise le composé de formule - (IV) pour obtenir la méthyl-4 2-H, 5-H pyrido [3', 4' : 4, 5] pyrrolo [3,2-c] pyridone-1 de formule (V)

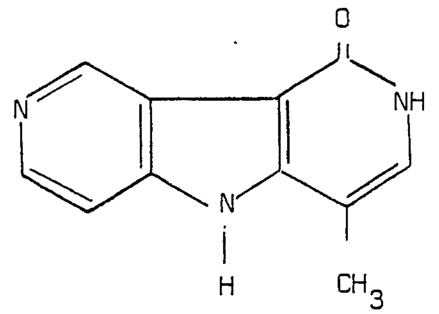

(V)

5°) on effectue la chloration du composé de formule (V) et on obtient la chloro-1 méthyl-4 5-H pyrido [3', 4' : 4, 5] pyrrolo [3,2-c] pyridine de formule (I) dans laquelle $R_1$ représente l'hydrogène :

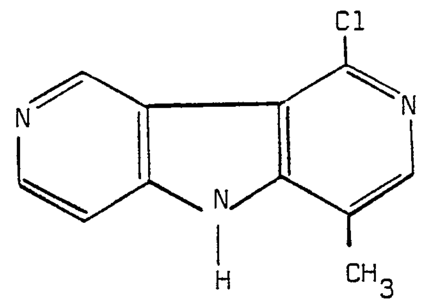

(I)

6°) et, le cas échéant, par action d'un halogène $ROCH_2X$, R étant un groupe alcoyle en $C_1$-$C_4$ ou un groupe alcoyl ($C_1$-$C_4$)-oxyalcoyle en $C_1$-$C_4$ et X un halogène, on obtient le dérivé quaternisé (VI) qui, en milieu basique, est transformé en anhydrobase (VII), de formules

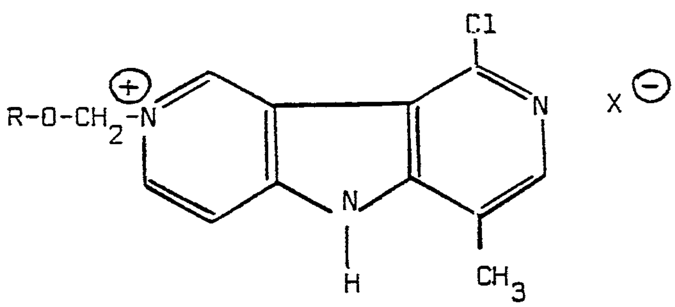

(VI)

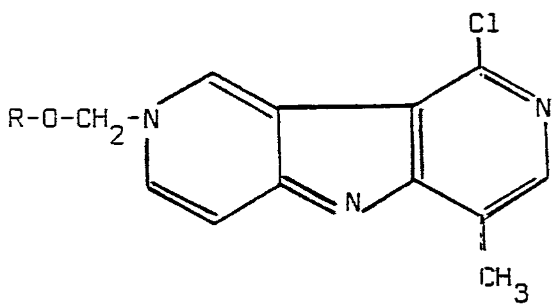

(VII)

dans lesquelles R et X sont comme ci-dessus.

Par action d'un halogénure ou d'un sulfate d'alcoyle $R_1$-Y, on obtient le dérivé quaternisé (VIII) alcoylé en position-5 qui par hydrolyse en milieu acide conduit à l'alcoyl -5 chloro-1 méthyl-4 5-H pyrido [3', 4' : 4,5] pyrrolo [3,2-c] pyridine de formule I dans laquelle $R_1$ représente le groupe alcoyle.

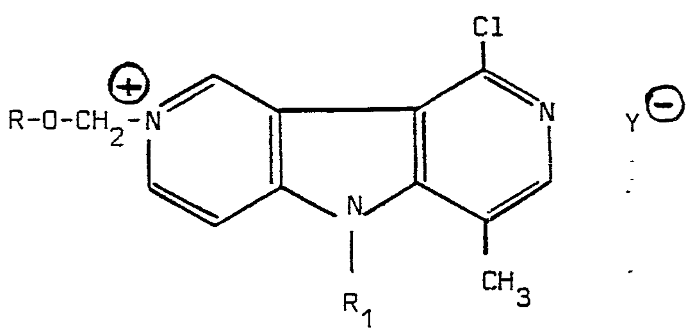

(VIII)

(dans laquelle R et $R_1$ sont comme ci-dessus)

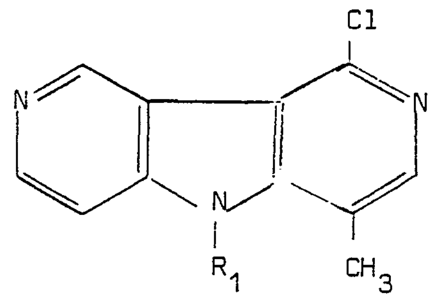

(I)

Par condensation, sur les composés de l'invention de formule (I), de l'amine de formule

$$NH_2 - (CH_2)_n - N \begin{matrix} R_2 \\ R_3 \end{matrix}$$

dans laquelle n, $R_2$ et $R_3$ ont les significations précitées, on obtient le composé de formule (A). Le produit de départ, l'hydroxy-4 méthyl-5 1-H pyridone-2 est un composé connu dont la synthèse a été décrite (E. BISAGNI, N.C. HUNG, Synthesis, 1984, p. 765).

La formation du composé de formule (II) s'effectue à une température comprise entre 100°C et 150°C, au reflux, dans un solvant inerte tel que l'éther mono éthylique de l'éthylène glycol. La formation du composé de formule (III) est effectuée rapidement au reflux d'éthanol absolu.

La cyclisation du composé de formule (III) est obtenue par chauffage à des températures comprises entre 200°C et 280°C au reflux d'un solvant inerte tel que le diphényl éther.

L'aromatisation du composé de formule (IV) qui conduit au composé de formule (V), s'effectue dans le même solvant que celui mis en oeuvre dans l'étape précédente, à haute température, en présence d'un catalyseur tel que le charbon palladié. Il n'est donc pas utile, dans cette opération, d'isoler le composé de formule (IV) intermédiaire, l'aromatisation s'effectuant simplement par l'introduction dans le milieu réactionnel du catalyseur, l'ensemble des deux réactions -cyclisation et aromatisation-étant de préférence réalisé en atmosphère inerte.

La chloration du composé de formule (V), obtenue par l'action du dichlorure de l'acide phényl phosphonique ou de l'oxychlorure de phosphore - ($POCl_3$) à une température comprise entre 100°C et 200°C, conduit au dérivé de formule (I) dans lequel $R_1$ représente l'hydrogène.

La quaternisation de ce dérivé s'effectue en milieu polaire aprotique tel que le N,N-diméthyl formamide et dès la température ambiante. Le passage à l'anhydrobase est avantageusement réalisé en milieu aqueux par action d'une solution d'hydroxyde de sodium environ 5 N et à une température comprise entre 20 et 80°C.

L'alcoylation du composé précédent s'effectue en milieu polaire aprotique, tel que l'acétone ou le N,N-diméthyl formamide, à une température comprise entre 40 et 110°C. Enfin la coupure du groupe alcoyloxyméthyle est réalisée, par exemple, en milieu aqueux sous l'action d'une solution d'un acide fort environ 5 N et à une température comprise entre 50 et 100°C.

Les exemples non limitatifs suivants sont donnés à titre d'illustration de l'invention :

EXEMPLE 1

Préparation de la chloro-1 méthyl-4 5-H pyrido [3', 4' : 4, 5] pyrrolo [3,2-c] pyridine (composé de formule (I) dans lequel $R_1$ = H)

A) hydrazino-4 méthyl-5 1-H pyridone-2 (composé de formule (II))

Le mélange formé par l'hydroxy-4 méthyl-5-1H pyridone-2 (16 g), l'éther monoéthylique de l'éthylène glycol (400 ml) et l'hydrate d'hydrazine - (140 ml) est chauffé au reflux pendant 4 jours et évaporé à sec sous pression réduite. Le résidu solide est repris dans 500 ml d'éthanol absolu

bouillant, filtré et le filtrat est concentré de moitié. Aprés une nuit à la température ambiante, le solide cristallisé est essoré et séché. On obtient des cristaux incolores (13 g) correspondant à l'hydrate du composé recherché F = 135-155°C.

Calc. pour $C_6H_9N_3O,H_2O$ = C, 45,85 ; H, 7,05 ; N,26,74.

Trouvé : C, 45,29 ; H, 6,97 ; N, 26,03.

Les eaux mères concentrées à 100 ml et abandonnées une nuit à la température ambiante fournissent une nouvelle quantité du composé (2,4 g). Le rendement total s'élève donc à 15,4 g, soit 76 p. 100.

RMN H, $[(CD_3)_2 SO]$ ; δ : 1,84 (d, 3H, $CH_3$, $J_{CH_3-H-6}$ = 1 Hz ; 4,06 (m, 2H, $NH_2$) ; 5,57 (s, 1H, H-3) ; 6,81 (d, 1H, H-6) ; 7,04 (s, 1H, -N H -$NH_2$) ; 10,13 - (s, 1H, NH-1).

B) Acétyl-1 [dihydro-1,2 méthyl-5 oxo-2 pyridyl-4] hydrazono]-4 pipéridone (compose de formule (III))

Le mélange formé par l'hydrazine (II) (15 g, 10,7 moles), la N-acétyl-pipéridone-4 (18g, 15,9 mmoles) dans l'éthanol absolu (400 ml) est chauffé à reflux pendant 1 h 30, laissé 15 h à la température ambiante et refroidi à 0° pour donner un premier précipité qui est filtré et séché. Les eaux mères, concentrées à 150 ml et laissées 15 h à la température ambiante fournissent une nouvelle quantité de cristaux. Au total, il se forme ainsi 24 g (84 p. 100) de microcristaux incolores correspondant à l'hydrate de l'hydrazone - F > 260°C.

Calc. pour $C_{13}H_{18}N_4O_2$, $H_2O$ : C, 55,70 ; H, 7,19 ; N, 19,99.

Trouvé : C, 56,04 ; H, 7,15 ; N, 19,62

C) Tétrahydro-6,7,8,9 méthyl-4 acétyl-8-2H,5H-pyrido [3', 4' : 4,5] pyrrolo [3,2-c] pyridone-1 (composé de formule (IV))

Le composé précédemment obtenu (2 g) est ajouté au diphényl éther (70 ml) et le mélange, placé sous atmosphère d'azote, est chauffé à reflux. Le solide se dissout peu à peu, le mélange réactionnel passe par une phase presque homogène puis un précipité réapparait progressivement. Le composé de départ est totalement transformé en 25 min. Le xylène (140 ml) est ajouté au mélange refroidi et le précipité formé est repris dans l'éthanol bouillant et filtré. Après addition de xylène (250 ml) le précipité obtenu est essoré. La concentration des eaux mères, jusqu'à élimination de tout l'éthanol, fournit, après refroidissement, une nouvelle quantité du produit attendu. Au total, il se forme ainsi 1,66 g (88,7 p. 100) de microcristaux incolores, partiellement hydratés.

F > 260°C

Calc. pour $C_{13}H_{15}N_3O_2$, 0,25 $H_2O$ : C, 62,52 ; H, 6,21 ; N, 16,83

Trouvé : C, 62,78 ; H, 6,27 ; N, 16,52.

RMN H, $[(CD_3)_2 SO]$ ; δ 2,11 et 2,14 (2s, 2 x 3H, $CH_3$-4 et $CH_3$-CO-) ; 2,53-2,9 (m, 2H, $CH_2$-6) ; 3,78 - (q, 2H, $CH_2$-7) ; 4,72 (s, 2H, $CH_2$-9) ; 6,72 (s, 1H, H-3) ; 10,98 (s, 1H, NH-2) ; 11,22 (s, 1H, NH-5).

D) Méthyl-4-2H, 5H-pyrido [3',4' : 4, 5] pyrrolo [3,2-c] pyridone-1 (composé de formule (V))

METHODE A Le composé précédemment obtenu (120 mg) est chauffé pendant 2 h 30 dans le diphényléther (13 ml) à reflux, en présence de 100 mg de charbon palladié à 10 p. 100. On ajoute encore 100 mg de charbon palladié, chauffe de nouveau à reflux pendant 1 h 30, laisse refroidir, ajoute 50 ml d'éthanol bouillant et filtre. Le résidu de l'évaporation du filtrat est repris dans l'éther de pétrole et le solide obtenu est recristallisé dans le minimum d'éthanol pour donner 60 mg (56,4 p. 100) de microcristaux incolores correspondant à l'hydrate du composé recherché. F > 270°C.

METHODE B Le mélange formé par l'hydrazone (III) (23,2 g) dans le diphényléther (1,4 l) est maintenu sous agitation sous atmosphère d'argon et chauffé à reflux pendant 30 min. On ajoute 4 g de charbon palladié à 30 p. 100 en suspension dans 100 ml de diphényléther et chauffe l'ensemble à reflux, sous agitation et sous argon, pendant 2 h. Après addition d'1 g de charbon palladié le chauffage est encore poursuivi pendant 1 h et le mélange réactionnel est refroidi. Après addition de 21 d'éthanol, l'ensemble est chauffé à reflux et filtré.

a) l'éthanol est évaporé sous pression réduite et le toluène (1,4 l) est ajouté au diphényl éther résiduel. Le précipité formé est essoré, repris dans 150 ml d'éthanol bouillant et refroidi pour donner 5,6 g du composé recherché.

b) le mélange solide filtré est repris dans 450 ml de diméthylformamide, filtré à l'ébullition, et le filtrat refroidi fournit 5,4 g du produit.

c) une nouvelle quantité (2 g) est obtenue après traitement de l'ensemble des eaux mères au charbon animal, filtration et concentration à 150 ml.

Par rapport au produit de départ (l'hydrazone) le rendement global calculé en produit pur, s'élève donc à 73,7 p. 100. F > 270°C.

Calc. pour : $C_{11}H_9O$, $H_2O$ : C, 60,82 ; H, 5,10 ; N, 19,35

Trouvé : C, 60,81 ; H, 5,11 ; N, 19,43.

RMN $H_1$ [$(CD_3)_2$ SO] ; δ 2,29 (d, 3H, $CH_3$-4) ; 7,21 (d, 1H, H-3, $J_{H_3\text{-}CH_3\text{-}4}$ = 1Hz) ; 7,49 (q, 1H, $J_{6\text{-}7}$ = 5,8 Hz, $J_{6\text{-}9}$ = 1 Hz) ; 8,38 (d, 1H, H-7) ; 9,26 (d, 1H, H-9) ; 11,10 (s, 1H, NH-5) ; 12 (s, 1H, NH-2).

E) Chloro-1 méthyl-4-5H-pyrido [3', 4' : 4, 5] pyrrolo [3,2-c] pyridine (composé de formule (I)).

Le mélange constitué par 500 mg du composé obtenu dans l'étape précédente (2,5 mmoles) et 25 ml de dichlorure de l'acide phényl phosphonique est chauffé au bain d'huile à 170°, sous agitation pendant 68 h et l'excès de dichlorure est évaporé à 130° sous 2 mm. L'eau (120 ml) est ajoutée au résidu et le mélange chauffé à l'ébullition puis filtré ; l'insoluble est lavé avec 40 ml d'eau bouillante et le filtrat alcalinisé, à froid, par addition d'ammoniaque. Le précipité formé est essoré, séché et recristallisé dans le xylène pour fournir 450 mg (82, 3 p. 100) de microcristaux incolores, F = > 270°C.

Calc. pour $C_{11}H_8ClN_3$ : C, 60,70; H, 3,70 ; N, 19,30 ; Cl, 16,29.

Trouvé : C, 60,50 ; H, 3,62 ; N 19,33 ; Cl, 16,11.

RMN $H_1$ [$(CD_3)_2$ SO) ;] δ : 2,57 (s, 3H, $CH_3$) ; 7,65 - (q, 1H, H-6, $J_{6\text{-}7}$ = 6Hz, $J_{6\text{-}9}$ = 1hz) ; 8,2 (s, 1H, H-3) ; 8,63 (d, 1H, H-7) ; 9,54 (d, 1H, H-9) ; 11,86 (s, 1H, NH).

EXEMPLE 2

Préparation de la Chloro-1 diméthyl-4,5-5-H pyrido [3', 4' : 4,5] pyrrolo [3,2-c] pyridine (composé de formule (I) dans lequel R = $CH_3$)

A) Chlorure de chloro-1 méthyl-4 [(méthyloxy-2 éthyl-1) oxyméthyl]-8 5-H pyrido [3', 4' : 4,5] pyrrolo (3',2-C] pyridinium (composé de formule (VI) dans lequel R = $CH_3$-O-$CH_2$-$CH_2$-).

La chloro-1 méthyl-4 5H pyrido [3', 4' : 4, 5] pyrrolo [3,2-c] pyridine (composé de formule (I) dans lequel $R_1$ = H) (18 g ; 0,08 mole) et le chlorométhyloxy-1 méthyloxy-2 éthane (12,5 g ; 0,1 mole) sont mis en suspension dans 200 ml de N,N-diméthyl formamide maintenus sous agitation pendant 3 heures à température ambiante. Le précipité formé est ensuite filtré, lavé àl'éther éthylique et séché.

Rendement : 92,6 % ; F = 206°C.

Calc. pour : $C_{15}$ $H_{17}$ $N_3$ $C_{12}$ $O_2$ : C, 52,64 ; H, 5,00 ;

N, 12,28 ; Cl, Trouvé : C, 52,11 ; H, 4,92 ;

N, 12,33 ; Cl,

RMN $H_1(D_{20})$ : δ : 2,20 (s, 3H, $CH_3$-4) ; 3,60 (s, 3H, O-$CH_3$) ; 3,87 -4,23 (m, 2 x 2H, O-$CH_2$-$CH_2$-O) ; 6,25 (s, 2H, O-$CH_2$-N) ; 7,64 (s, 1H, H-3) ; 8,10 (d, 1H, H-6, J 6-7 = 6 Hz) ; 9,0 (d, 1 H, H 7) 9,45 (s, 1 H, H-9).

B) Chloro-1 méthyl-4 [(méthyloxy-2 éthyl-1) oxyméthyl]-8 8-H pyrido [3', 4' : 4,5] pyrrolo [3,2-c] pyridine (composé de formule (VII) dans lequel R = $CH_3$-O-$CH_2$-$CH_2$-).

Le dérivé quaternisé précédent (25 g ; 0,73 mole) est dissous dans 200 ml d'eau. Puis on introduit 50 ml d'une solution aqueuse d'hydroxyde de sodium à 50 % et l'on chauffe le milieu à 50°C pendant 2 heures. L'huile obtenue est extraite au chlorure de méthylène. La phase organique est lavée à l'eau, séchée et concentrée sous pression réduite. Le précipité obtenu est recristallisé dans le toluène.

Rendement : 77 % ; F = 114°C.

Calc. pour : $C_{15}$ $H_{16}$ $N_3$ $C_{102}$ : C, 58,92 ; H, 5,27 ;

N, 13,73 ; Cl, 11,59 trouvé: C, 58,77 ; H, 5,13 ;

N, 13,79 ; Cl, 11,56.

RMN $H_1$ (CD $C_{13}$) : δ : 2,62 (s, 3H, $CH_3$-4) ; 3,33 (s, 3H, O-$CH_3$) ; 4,49 -4,75 (m,. 2 x 2H, O-$CH_2$-$CH_2$-O) ; 5,62 (s, 2H, -O$CH_2$-N) ; 7,60 (d, 1H, $H_6$, J 6-7 = 6 Hz); 7,82 (d, 1H, H-7) ; 8,15 (s, 1H, H-3) ; 8,87 (s, 1H, H-9).

C) Iodure de chloro-1 diméthyl-4,5 [(méthyloxy-2 éthyl-1) oxyméthyl]-8 5-H pyrido [3', 4' : 4,5] pyrrolo [3,2-c] pyridinium (composé de formule (VIII) dans lequel $R_1$ = $-CH_3$ et R = $CH_3-O-CH_2-CH_2-$).

La chloro-1 méthyl-4 [(méthyloxy-2 éthyl-1) oxyméthyl]-8 8-H pyrido [3', 4' : 4,5] pyrrolo [3,2-c] pyridine (17 g ; 0,055 mole) est dissous dans 150 ml d'acétone. On introduit alors l'iodure de méthyle (11,8 g ; 0,083 mole). Le milieu est ensuite chauffé dans un autoclave pendant 6 heures à 60°C. Après refroidissement, le précipité obtenu est filtré et lavé à l'acétone. On obtient ainsi 13 g de produit attendu. Les phases acétoniques sont ensuite rassemblées et concentrées sous pression réduite. Le résidu obtenu est repris par 30 ml d'acétone et refroidi à 4°C. Le second précipité obtenu est filtré pour obtenir 7 g de produit attendu. On isole ainsi 20 g de produit attendu brut qui contient déjà environ 25 % de chloro-1 diméthyl-4,5 5-H pyrido [3', 4' : 4,5] pyrrolo [3,2-c] pyridine.

Rendement : environ 83 % ; F environ 180°C - (décomposition).

RMN ($DMSOd_6$) : δ : 2,94 (s, 3H, $CH_3$-4) ; 3,27 (s, 3H, $O-CH_3$) ; 3,50-3,95 (m, 2 x 2H, $O-CH_2-CH_2-O$) ; 4,45 (s, 3H, $CH_3$-5) ; 6,20 (s, 2H-$O-CH_2-N$); 8,50 (s, 1H, H-3) ; 8,62 (d, 1H, H-6, J 6-7 = 7 Hz) ; 9,19 (d, 1H, H-7) ; 10,0 (s, 1H, H-9).

D) Chloro-1 diméthyl-4,5 5-H pyrido [3', 4' : 4,5] pyrrolo [3,2-c] pyridine.

Le dérivé quaternisé précédent brut (18 g ; 0,04 mole) est dissous dans 200 ml d'une solution aqueuse d'acide chlorhydrique 6 N. Le milieu est ensuite chauffé au reflux pendant 3 heures. Après refroidissement, le milieu est alcalinisé par une solution aqueuse d'hydroxyde de sodium 10 N. Le précipité obtenu est repris dans 200 ml de méthanol bouillant en présence de noir animal. La solution méthanolique est filtrée puis concentrée à pression réduite. Le produit brut ainsi obtenu est purifié par chromatographie sur colonne de silice - (éluant dichlorométhane/méthanol ; 9/1).

Rendement : 71 % ; F = 236°C.

Calc. pour $C_{12}H_{10}N_3Cl$ : C, 62,21 ; H, 4,35

N, 18,14 ; Cl, 15,30 ; C, 62,32 ; H, 4,34 ;

N, 18,34 ; Cl, 15,05.

RMN $H_1$ ($CDCl_3$) ; δ :2,8 (s, 3H, $CH_3$-4) ; 4,13 (s, 3H, $N-CH_3$) ;

7,34 (q, 1H, H-6, $J_{6-7}$ = 6Hz, $J_{6-9}$ = 1hz) ; 8,1 (s, 1H, H-3) ;

8,68 (d, 1H, H-7) ; 9,71 (d, 1H, H-9).

EXEMPLE 3

Préparation de la chloro-1 méthyl-4 éthyl-5 5-H pyrido [3', 4' : 4,5] pyrrolo [3,2-c] pyridine - (composé de formule (I) dans lequel $R_1$ = $C_2H_5$)

Ce composé est préparé selon la même méthode que dans l'exemple 2 à partir du composé de formule (I) R = H et de l'iodure d'éthyle dans l'étape C. Rendement de 41 p. 100 à partir du composé de formule (I) dans lequel R = H F = 167°-168°C.

RMNH, (($CDCl_3$) ; δ : 1,52 (t, 3H, <u>$CH_3$</u>-$CH_2$) ; 2,79 (s, 3H, $-CH_3$-4) ; 4,62 (q, 2H, -<u>$CH_2$</u>-$CH_3$) ; 7,45 - (d, 1H, H-6, J 6-7=6Hz) ; 8,18 (s, 1H, H-3) ; 8,71 - (d, 1H, H-7) ; 9,75 (s, 1H, H-9).

A titre illustratif, on décrit ci-dessous, la préparation de deux composés de formule A, à partir d'un composé de formule (I) : la [-(diéthylamino-3 propyl-1) amino]-1 méthyl-4 5-H pyrido [3', 4', : 4,5] pyrrolo [3,2-c] pyridine, Trimaléate ($R_1$ = H).

La chloro-1 méthyl-4 5-H pyrido [3' , 4' : 4,5] pyrrolo [3,2-c] pyridine de formule (I) ($R_1$ = H) - (1g) est chauffée sous atmosphère d'azote dans la diéthylamino-3 propyl amine (10 g) à 160-170°C pendant 18 h. en suivant la disparition du dérivé chloré par chromatographie sur couche mince - (silice, éluant : chlorure de méthylène-éthanol 9/1). Puis l'excès d'amine est éliminé sous pression réduite. Le résidu est repris dans 50 ml d'une solution d'hydroxyde de sodium 3N et extrait au chlorure de méthylène. La phase organique est séchée puis concentrée sous pression réduite. Le produit brut est chromatographié sur colonne de silice (chlorure de méthylène-éthanol 95/5). La base ainsi obtenue est dissoute dans une solution acétonique d'acide maléique (au moins 3 équivalents molaires) qui est maintenue à l'ébullition pendant deux minutes.

Après refroidissement le trimaléate précipite. Il est filtré et séché. Rendement 71 p. 100 ; F = 180°C avec décomposition.

Calc. pour $C_{30}H_{37}N_5O_{12}$ : C, 54,62 ; H, 5,65 ; N, 10,62

Trouvé : C, 54,28 ; H, 5,90 ; N, 10,51

La [(diéthylamino-3 propyl-1) amino]-1 diméthyl-4,5 5-H pyrido [3', 4' : 4,5] pyrrolo [3,2-c] pyridine, trichlorhydrate. ($R_1$ = $CH_3$). Ce composé a été préparé à partir de la chloro-1 diméthyl-4,5 5-H pyrido [3', 4' : 4, 5] pyrrolo [3,2-c] pyridine - (composé de formule (II) $R_1$ = $CH_3$) et de la diéthylamino-3 propylamine par chauffage pendant 24 h. Après chromatographie, la base est reprise par une solution éthanolique d'acide chlorhydrique. On obtient un précipité de trichlorhydrate qui est filtré et séché.

Rendement 64 p. 100 ; se sublime à partir de 200°C.

Calc. pour $C_{19}H_{27}N_5$ ; 3 HCl ; 3 $H_2O$ : C, 46,73 ; H, 7,37 ; N, 14,33 ; Cl, 21,80

Trouvé : C, 46,48 ; H, 7,81 ; N, 13,88 ; Cl, 22,08

RMN $H_1$ ($D_{2}0$) ; $\delta$ : 1,49 (t, 2X3H, -$CH_2$-$\underline{CH_3}$) ; 2,37 - (m, 2H, $CH_2$-$\beta$) ; 2,9 (d, 3H, $CH_3$-4) ; 3,23-3,56 (m, 3X2H, -$\underline{CH_2}$-$CH_3$ + $CH_2$ -$\gamma$) ; 3,91 (t, 2H, $CH_2$-$\alpha$); 4,46 (s, 3H, N-$CH_3$) ; 7,99 (d, 1H, H-3, $J_3$ -$CH_3$-4 = 1Hz) ; 8,35 (d, 1H, H-6, J 6-7 = 7 Hz) 8,87 (d, 1H, H-7) ; 9,87 (s, 1H, H-9).

## Revendications

1) -Intermédiaires de synthèse de formule

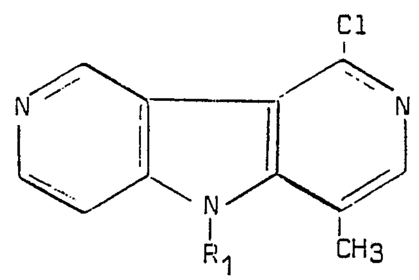

dans laquelle $R_1$ représente l'hydrogène ou un radical alcoyle inférieur linéaire ou ramifié de 1 à 4 atomes de carbone, ainsi que les formes tautomères lorsqu'elles existent.

2) -chloro-1 méthyl-4 5-H pyrido [3', 4' : 4,5] pyrrolo [3,2-c] pyridine

3) -Chloro-1 diméthyl-4,5-5-H pyrido [3', 4' : 4,5] pyrrolo [3,2-c] pyridine

4) -Chloro-1 méthyl-4 éthyl-5 5-H pyrido [3', 4' : 4,5] pyrrolo [3,2-c] pyridine.

5) -Procédé pour la préparation des composés de formule (I) caractérisé en ce que

a) par réaction de l'hydrazine hydratée sur l'hydroxy-4 méthyl-5 1-H pyridone, on prépare l'hydrazino-4 pyridone-2 de formule (II)

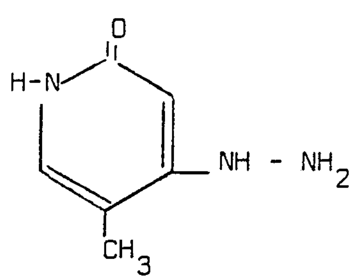

(II)

b) on fait réagir l'hydrazine de formule (II) sur la N-acétyl pipéridone-4 pour obtenir l'hydrazone correspondante de formule (III)

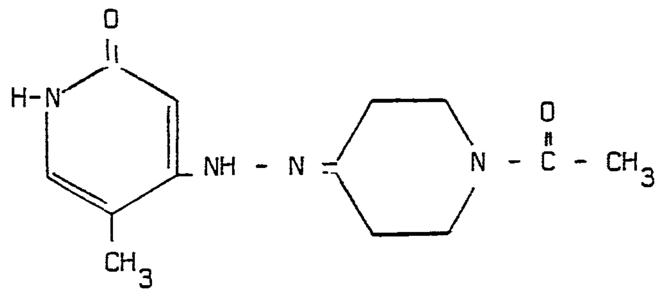

(III)

c) par la réaction de Fisher, on cyclise l'hydrazone de formule (III) pour former la tétrahydro-6,7,8,9 acétyl-8 méthyl-4 2-H,5-H pyrido [3', 4' : 4,5] pyrrolo [3,2-c] pyridone-1 de formule (IV)

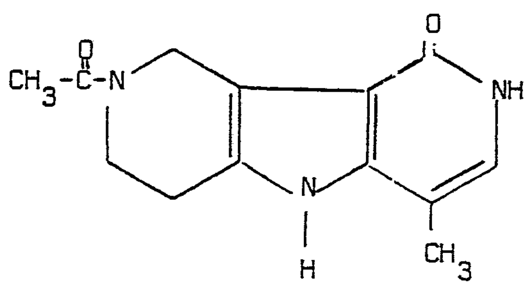

(IV)

d) on aromatise le composé de formule (IV) pour obtenir la méthyl-4 2-H, 5-H pyrido [3', 4' : 4,5] pyrrolo [3,2-c] pyridone-1 de formule (V)

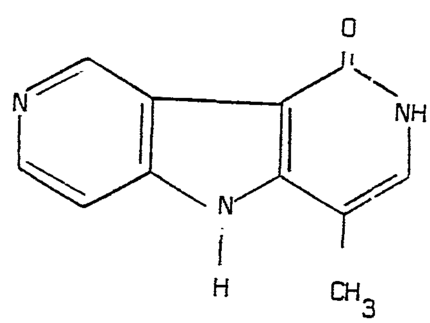

(V)

e) par chloration du composé de formule (V), on obtient la chloro-1 méthyl-4 5-H pyrido [3', 4' : 4,5] pyrrolo [3,2-c] pyridine de formule (I) dans laquelle $R_1$ = H

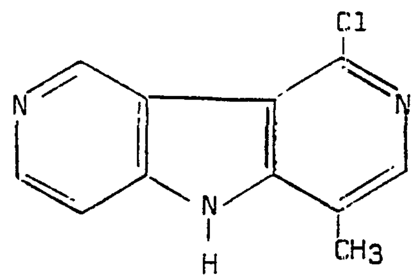

(I)

f) et, le cas échéant, par action d'un halogénure $ROCH_2X$, R étant un groupe alcoyle en $C_1$-$C_4$ ou un groupe alcoyl ($C_1$-$C_4$) oxyalcoyle en $C_1$-$C_4$ et X un halogène, sur le composé de formule (I) dans laquelle $R_1$ = H, on obtient un halogénure d'alcoyloxyméthyl-8 chloro-1 méthyl-4 5-H pyrido /3', 4' : 4,5/pyrrolo /3,2-c/ pyridinium de formule VI :

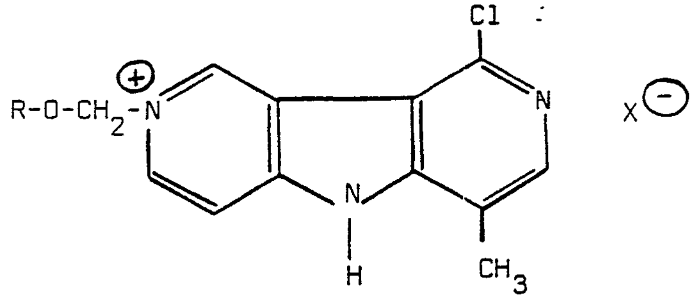

(VI)

dans laquelle R et X sont comme ci-dessus ;

g) par action d'une base forte sur le composé de formule (VI), on obtient une alcoyloxyméthyl-8 chloro-1 méthyl-4 8-H pyrido [3', 4' : 4,5] pyrrolo [3,2-c] pyridine de formule (VII)

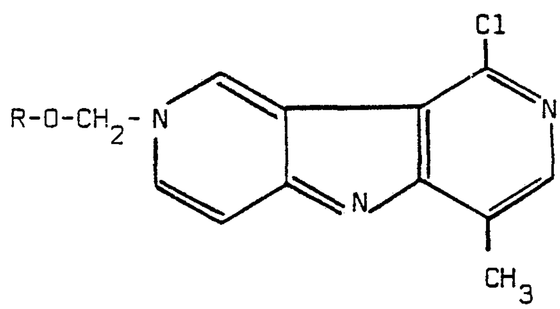

(VII)

h) par action d'un halogénure (ou d'un sulfate) d'alcoyle $R_1$-Y sur un composé de formule (VII), on obtient un halogénure (ou un sulfate) d'alcoyl-5 alcoyloxyméthyl-8 chloro-1 méthyl-4 5-H pyrido [3', 4' : 4,5] pyrrolo [3,2-c] pyridine de formule (VIII)

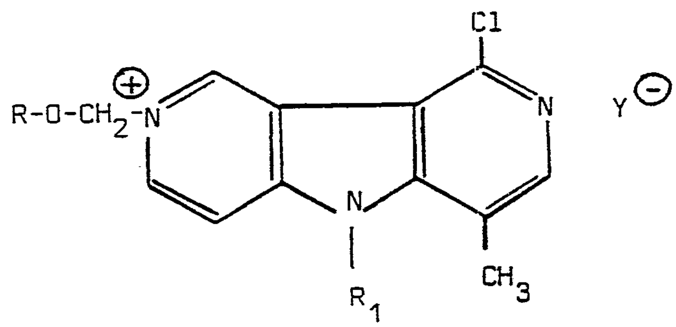

(VIII)

dans laquelle R et $R_1$ sont comme ci-dessus, et

i) par action d'une solution aqueuse d'acide fort sur un composé de formule (VIII), on obtient un alcoyl-5 chloro-1 méthyl-4 5-H pyrido [3', 4' : 4,5] pyrrolo [3,2-c] pyridine de formule (I) dans laquelle $R_1$ = alcoyle.

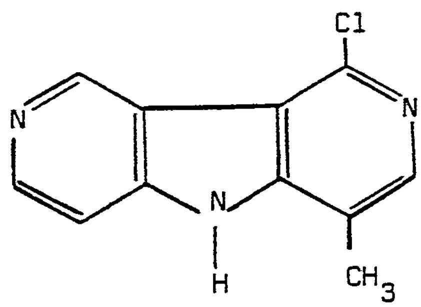

(I)

6) Procédé selon la revendication 5 caractérisé en ce que la cyclisation du composé de formule (III) et l'aromatisation du composé de formule - (IV) sont effectuées dans le même solvant sans isolement du dérivé intermédiaire de formule - (IV).

Revendications pour l'Etat contractant : AT

1. Procédé pour la préparation des composés de formule (I)

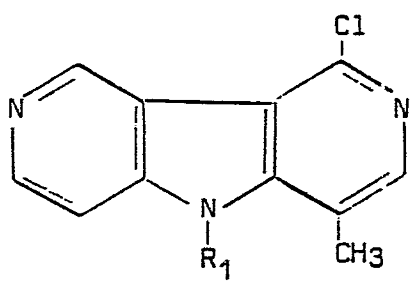

(I)

dans laquelle $R_1$ représente l'hydrogène ou un radical alcoyle inférieur linéaire ou ramifié de 1 à 4 atomes de carbone, ainsi que les formes tautomères lorsqu'elles existent, caractérisé en ce que

a) par réaction de l'hydrazine hydratée sur l'hydroxy-4 méthyl-5 1-H pyridône, on prépare l'hydrazino-4 pyridone-2 de formule (11)

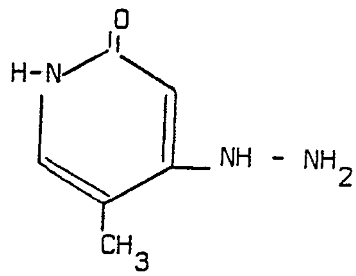

(II)

b) on fait réagir l'hydrazine de formule (II) sur la N-acétyl pipéridone-4 pour obtenir l'hydrazone correspondante de formule (III)

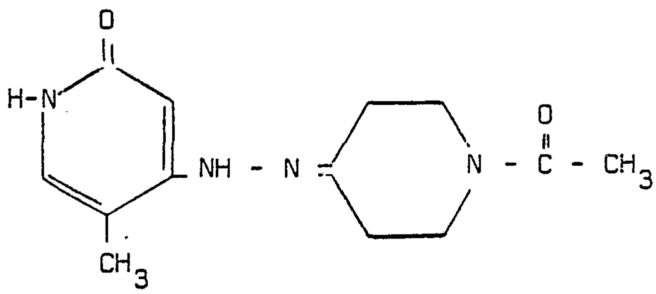

(III)

c) par réaction de Fisher, on cyclise l'hydrazone de formule (III) pour former la tétrahydro-6,7,8,9 acétyl-8 méthyl-4 2-H,5-H pyrido [3', 4' : 4,5] pyrrolo [3,2-c] pyridone-1 de formule (IV)

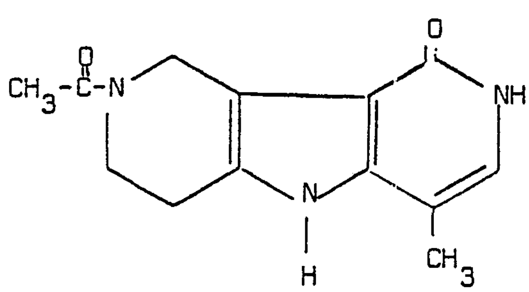

(IV)

d) on aromatise le composé de formule (IV) pour obtenir la méthyl-4 2-H, 5-H pyrido [3', 4' : 4,5] pyrrolo [3,2-c] pyridone-1 de formule (V)

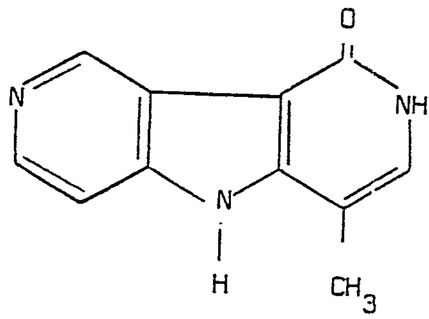

(V)

e) par chloration du composé de formule (V), on obtient la chloro-1 méthyl-4 5-H pyrido [3', 4' : 4,5] pyrrolo [3,2-c] pyridine de formule (I) dans laquelle $R_1$ = H

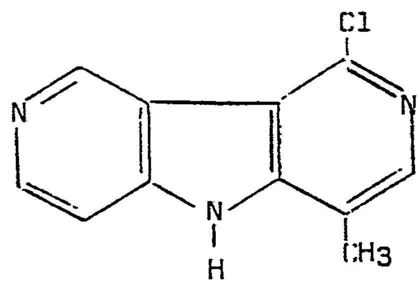

(I)

f) et, le cas échéant, par action d'un halogénure $ROCH_2X$, R étant un groupe alcoyle en $C_1$-$C_4$ ou un groupe alcoyl ($C_1$-$C_4$) oxyalcoyle en $C_1$-$C_4$ et X un halogène, sur le composé de formule (I) dans laquelle $R_1$ = H, on obtient un halogénure d'alcoyloxyméthyl-8 chloro-1 méthyl-4 5-H pyrido /3', 4' : 4,5/pyrrolo /3,2-c/ pyridinium de formule VI :

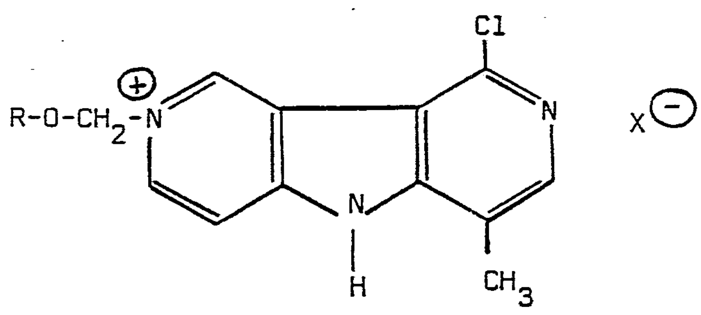

(VI)

dans laquelle R et X sont comme ci-dessus ;

g) par action d'une base forte sur le composé de formule (VI), on obtient une alcoyloxyméthyl-8 chloro-1 méthyl-4 8-H pyrido [3', 4' : 4,5] pyrrolo [3,2-c] pyridine de formule (VII)

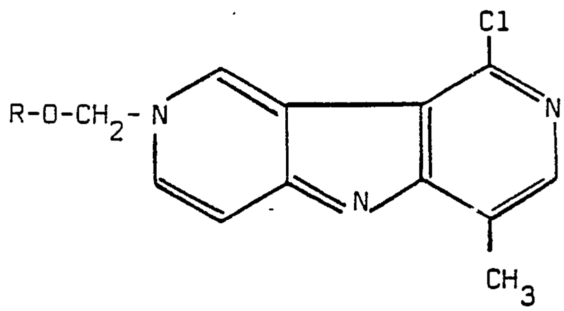

(VII)

h) par action d'un halogénure (ou d'un sulfate) d'alcoyle $R_2$-Y sur un composé de formule (VII), on obtient un halogénure (ou un sulfate) d'alcoyl-5 alcoyloxyméthyl-8 chloro-1 méthyl-4 5-H pyrido [3', 4' : 4,5] pyrrolo [3,2-c] pyridine de formule (VIII)

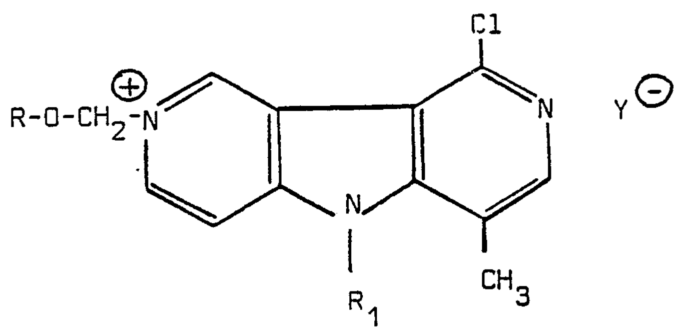

(VIII)

dans laquelle R et $R_1$ sont comme ci-dessus, et

i) par action d'une solution aqueuse d'acide fort sur un composé de formule (VIII), on obtient un alcoyl-5 chloro-1 méthyl-4 5-H pyrido [3', 4' : 4,5] pyrrolo [3,2-c] pyridine de formule (I) dans laquelle $R_1$ = alcoyle.

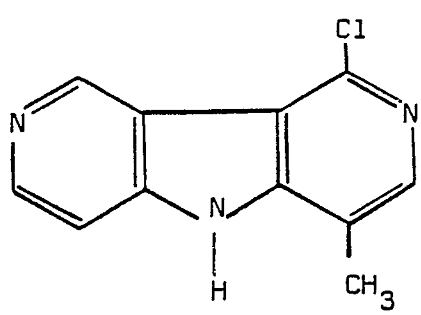

(I)

6) Procédé selon la revendication 1, caractérisé en ce que la cyclisation du composé de formule (III) et l'aromatisation du composé de formule -(IV) sont effectuées dans le même solvant sans isolement du dérivé intermédiaire de formule -(IV).

Office européen des brevets

**RAPPORT DE RECHERCHE EUROPEENNE**

Numéro de la demande

EP 86 40 0602

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int. Cl.4) |
| | Néant<br>----- | | C 07 D 471/14 //<br>(C 07 D 471/14<br>C 07 D 221:00<br>C 07 D 221:00<br>C 07 D 209:00) |
| | | | DOMAINES TECHNIQUES RECHERCHES (Int. Cl.4) |
| | | | C 07 D 471/00 |

Le présent rapport de recherche a été établi pour toutes les revendications

| Lieu de la recherche | Date d'achèvement de la recherche | Examinateur |
|---|---|---|
| LA HAYE | 27-06-1986 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82